# EUROPEAN PATENT SPECIFICATION

(11) **EP 1 763 356 B1**
(45) Date of publication and mention of the grant of the patent: **16.07.2008**
(21) Application number: 05738307.7
(22) Date of filing: 15.04.2005
(51) Int. Cl.: A61K 31/7088, A61K 31/7068, A61P 35/00

(54) **COMPOSITION COMPRISING A SURVIVIN ANTISENSE OLIGONUCLEOTIDE AND GEMCITABINE FOR THE TREATMENT OF CANCER**
ZUSAMMENSETZUNG MIT EINEM SURVIVIN-ANTISENSE-OLIGONUCLEOTID UND GEMCITABIN ZUR BEHANDLUNG VON KREBS
COMPOSITION COMPRENANT UN OLIGONUCLEOTIDE SURVIVIN ANTISENS ET GEMCITABINE POUR LE TRAITEMENT DU CANCER

(30) Priority: 22.04.2004 US 564540 P
(43) Date of publication of application: 21.03.2007
(60) Divisional application: 07119706.5
(73) Proprietor: ELI LILLY AND COMPANY, Indianapolis, Indiana 46285 (US)
(72) Inventor: PATEL, Bharvin, Kumar, Westfield, IN 46074 (US)
(74) Representative: Burnside, Ivan John
(86) International application number: PCT/US2005/012968
(87) International publication number: WO 2005/107771

(56) References cited:
- WO-A-20/04069991
- US-A- 5 464 826
- US-A- 6 077 709
- US-A1- 2002 137 708
- US-B1- 6 335 194
- FUESSEL ET AL: "326 - Chemosensitization of bladder cancer cells by survivin-directed antisense oligodeoxynucleotides and sirna" EUROPEAN UROLOGY SUPPLEMENTS, XX, XX, vol. 4, no. 3, March 2005 (2005-03), page 84, XP005006790 ISSN: 1569-9056
- HOLMLUND J T: "APPLYING ANTISENSE TECHNOLOGY AFFINITAK AND OTHER ANTISENSE OLIGONUCLEOTIDES IN CLINICAL DEVELOPMENT" ANNALS OF THE NEW YORK ACADEMY OF SCIENCES, NEW YORK ACADEMY OF SCIENCES, NEW YORK, NY, US, vol. 1002, 2003, pages 244-251, XP008035612 ISSN: 0077-8923
- KAMI ET AL: "A novel combined therapy with oncolytic replication-competent HSV-1 vector plus gemcitabine for pancreatic cancer" JOURNAL OF SURGICAL RESEARCH, ACADEMIC PRESS INC., SAN DIEGO, CA, US, vol. 130, no. 2, February 2006 (2006-02), page 307, XP005265561 ISSN: 0022-4804

## Description

This invention relates to the use of compound I, an antisense oligonucleotide therapeutic directed to Survivin, in conjunction with the chemotherapeutic agent gemcitabine hydrochloride in order to enhance the effectiveness of the chemotherapeutic agent.

### BACKGROUND OF THE INVENTION

Survivin is a protein in the inhibitor of apoptosis (IAP) family that regulates apoptosis and cytokinesis. Survivin is frequently overexpressed in many forms of cancer, but is largely absent in the normal adjacent tissue. Expression level often correlates with decreased apoptosis in tumors. To date, overexpression of survivin has been detected in tumors of the lung, colon, pancreas, prostate, breast, stomach, and in non-Hodgkin's lymphoma, and neuroblastoma (Altieri, Nature Cancer Rev., 3: 46-54 (2002); Adida et al., Lancet 351:882-883 (1998); Ambrosini et al., Nat. Med. 3: 917-921 (1997); Lu et al., Cancer Res. 58:1808-1812 (1998)).

U.S. Patents 6,077,709 and 6,165,788 disclose antisense compounds, particularly antisense oligonucleotides (ASOs), and methods for modulating expression or overexpression of survivin. These compounds are useful for the treatment of cancer, including, but not limited to pancreatic cancer, prostate cancer, colon cancer, breast cancer, lung cancer, bladder cancer, stomach cancer, neuroblastoma, non-Hodgkin's lymphoma, and cancer involving keratinocyte or fibroblast cells. U.S. Patent 6,335,194 discloses the use of antisense oligonucleotides directed to survivin in combination with chemotherapeutic agents that work by a mechanism distinct from the antisense mechanism.

Blocking the expression of survivin with an antisense oligonucleotide directed to survivin will restore default cell-death checkpoints and, either in itself or in combination with chemotherapy, selectively eliminate cancer cells and improve clinical outcome.

The present invention describes the discovery that susceptible neoplasms may be treated in an advantageous or superior manner by combination therapy using a survivin antisense oligonucleotide in combination with an additional anti-cancer agent.

### BRIEF SUMMARY OF THE INVENTION

The present invention provides compositions useful for treating cancer, including hepatocellular cancer, pancreatic cancer, prostate cancer, colon cancer, breast cancer, lung cancer, bladder cancer, stomach cancer, neuroblastoma, non-Hodgkin's lymphoma, and cancer involving keratinocyte or fibroblast cells. The present invention includes the use of an antisense oligonucleotide for blocking the expression or overexpression of survivin, in combination with gemcitabine HCl, which is described in US Patent 5,464,826.

Surprisingly, we have found that the combination of an antisense oligonucleotide that inhibits the expression or overexpression of Survivin with certain additional anti-cancer agents results in a greater-than-additive inhibition of tumor volume compared to treatment with either agent alone.

The types of cancers that may be treated with the compositions and methods of the present invention include the following: neoplasms of the central nervous system: glioblastoma multiforme, astrocytoma, oligodendroglial tumors, ependymal and choroid plexus tumors, pineal tumors, neuronal tumors, medulloblastoma, schwannoma, meningioma, meningeal sarcoma; neoplasms of the eye: basal cell carcinoma, squamous cell carcinoma, melanoma, rhabdomyosarcoma, retinoblastoma; neoplasms of the endocrine glands: pituitary neoplasms, neoplasms of the thyroid, neoplasms of the adrenal cortex, neoplasms of the neuroendocrine system, neoplasms of the gastroenteropancreatic endocrine system, neoplasms of the gonads; neoplasms of the head and neck: head and neck cancer, oral cavity, pharynx, larynx, odontogenic tumors; neoplasms of the thorax: large cell lung carcinoma, small cell lung carcinoma, non-small cell lung carcinoma, neoplasms of the thorax, malignant mesothelioma, thymomas, primary germ cell tumors of the thorax; neoplasms of the alimentary canal: neoplasms of the esophagus, neoplasms of the stomach, neoplasms of the liver, neoplasms of the gallbladder, neoplasms of the exocrine pancreas, neoplasms of the small intestine, veriform appendix and peritoneum, adneocarcinoma of the colon and rectum, neoplasms of the anus; neoplasms of the genitourinary tract: renal cell carcinoma, neoplasms of the renal pelvis and ureter, neoplasms of the bladder, neoplasms of the urethra, neoplasms of the prostate, neoplasms of the penis, neoplasms of the testis; neoplasms of the female reproductive organs: neoplasms of the vulva and vagina, neoplasms of the cervix, addenocarcinoma of the uterine corpus, ovarian cancer, gynecologic sarcomas; neoplasms of the breast; neoplasms of the skin: basal cell carcinoma, squamous cell carcinoma, dermatofibrosarcoma, Merkel cell tumor; malignant melanoma; neoplasms of the bone and soft tissue: osteogenic sarcoma, malignant fibrous histiocytoma, chondrosarcoma, Ewing's sarcoma, primitive neuroectodermal tumor, angiosarcoma; neoplasms of the hematopoietic system: myelodysplastic sydromes, acute myeloid leukemia, chronic myeloid leukemia, acute lymphocytic leukemia, HTLV-1 and T-cell leukemia/lymphoma, chronic lymphocytic leukemia, hairy cell leukemia, Hodgkin's disease, non-Hodgkin's lymphomas, mast cell leukemia, and neoplasms of children: acute lymphoblastic leukemia, acute myelocytic leukemias, neuroblastoma, bone tumors, rhabdomyosarcoma, lymphomas, renal tumors.

Of the indications above, pancreatic cancer, colorectal cancer, hepatocellular cancer, bladder cancer, lung cancer, breast cancer, ovarian cancer, and lymphoma, such as non-Hodgkin's lymphoma, are preferred disorders to be treated by the compositions of the present invention. The compositions of the present invention are more preferred for the treatment of hepatocellular cancer, bladder cancer, colorectal cancer, pancreatic cancer, and non-Hodgkin's lymphoma. The compositions and methods of the present invention are especially useful for the treatment of hepatocellular cancer.

Thus, the present invention relates to the treatment of a susceptible neoplasm comprising separate administration of a Survivin ASO and an additional anti-cancer agent.

In a further embodiment, the present invention relates to the treatment of a susceptible neoplasm comprising simultaneous administration of a Survivin.ASO and an additional anti-cancer agent.

That is, the present invention provides for administration of a Survivin ASO and an additional anti-cancer agent for the treatment of susceptible neoplasms.

In another aspect, the present invention provides for the use of a Survivin ASO in combination with an additional anti-cancer agent in the manufacture of a medicament for treating susceptible neoplasms by means of the method described above.

Thus, in another embodiment, the present invention relates to a pharmaceutical composition comprising a Survivin ASO and an additional anti-cancer agent. That is, the present invention provides a composition comprising a Survivin ASO and an additional anti-cancer agent, for use in therapy.

### DETAILED DESCRIPTION OF THE INVENTION

In the methods of the present invention, the target RNA, target gene, or other target genomic polynucleotide region is that of Survivin. As used herein, the terms "target nucleic acid" and "nucleic acid encoding Survivin" encompass DNA encoding survivin, RNA (including pre-mRNA and mRNA or portions thereof) transcribed from such DNA, and also cDNA derived from such RNA. The specific hybridization of an oligomeric compound with its target nucleic acid interferes with the normal function of the nucleic acid. This modulation of function of a target nucleic acid by compounds that specifically hybridize to it is generally referred to as "antisense".

The term "Survivin Antisense Oligonucleotide (Survivin ASO)" refers to a modified or unmodified compound that is at least 70% homologous with the following sequence: 5'-TGTGCTATTCTGTGAATT-3'. Preferably, the modified or unmodified compound is at least 80% homologous with the following sequence: 5'-TGTGCTATTCTGTGAATT-3'. Still more preferably, the modified or unmodified compound is at least 90%, or more preferably, 95% homologous with the following sequence: 5'-TGTGCTATTCTGTGAATT-3'. Most preferably, the modified or unmodified compound is at least 99% homologous with the following sequence: 5'-TGTGCTATTCTGTGAATT-3'. More particularly, the term "Survivin ASO" refers to a modified compound that is at least 80%, 90%, 95%, or 99% homologous with the above sequence wherein every internucleoside linkage is a phosphorothioate linkage. Still more particularly, the modified compound that is at least 80%, 90%, 95%, or 99% homologous with the above sequence wherein every internucleoside linkage is a phosphorothioate linkage further comprises a 2'-methoxyethoxy modification at the four bases at the 5' end and the four bases at the 3' end. Still more particularly, the cytosine residues of the modified compound comprise a 5-methyl modification. The term "Survivin ASO" most particularly refers to a compound of the following sequence: 5'-TGTGCTATTCTGTGAATT-3', wherein every internucleoside linkage is a phosphorothioate linkage, four bases at the 5' end comprise a 2'-methoxyethoxy modification, four bases at the 3' end comprise a 2'-methoxyethoxy modification, and the cytosine residues at positions 5 and 10 comprise a 5-methyl modification. Even more particularly, the term "Survivin ASO" refers to the sodium salt of the above modified compound. Most preferably, the term "Survivin ASO" refers to the following compound.

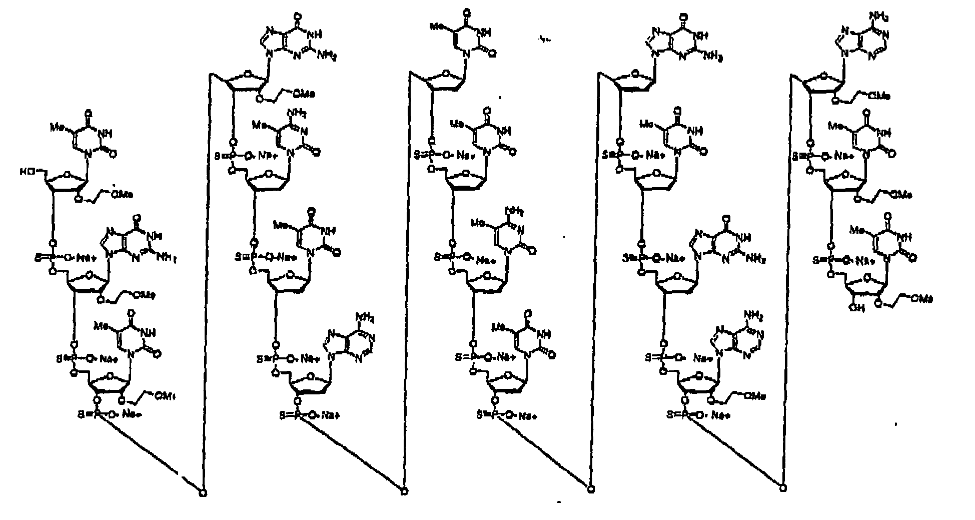

Paclitaxel is a chemotherapeutic agent isolated from the Pacific yew tree *Taxis brevifolia* and a member of the taxane family of terpenes (Wani et al. (1971) J. Am. Chem. Soc. 93: 2325). A review of the synthesis and anticancer activity of paclitaxel derivatives is found in Kingston et al., Studies in Organic Chemistry, vol. 26, "New Trends in Natural Products Chemistry 1986," Attaur-Rahman and Le Quesne, Eds. (Elsevier, Amsterdam, 1986) pages 219-235.

Gemcitabine HCl, a nucleoside analogue that exhibits antitumor activity, is 2'-deoxy-2',2'-difluorocytidine monohydrochloride (β-isomer), also known as 2',2'-difluoro-2'-deoxycytidine monohydrochloride, or as 1-(4-amino-2-oxo-1H-pyrimidin-1-yl)-2-desoxy-2',2'-difluororibose. The structural formula is depicted below.:

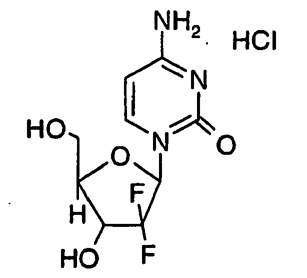

Gemcitabine HCl is described in US Patent 5,464,826 which discloses the methods of preparing the compound, formulating the compound, and the treatment of cancer using the compound.

Doxorubicin HCl is an anthracycline antibiotic that has been widely used as an anti-tumor agent. It is isolated from the fungus *Streptomyces peucetius* var. *caesius.* Doxorubicin HCl may also be synthesized by procedures well known in the art. Literature references include: Henry, D.W., ACS Symposium Series, No. 30, Cancer Chemotherapy, American Chemical Society (1976) pages 15-57; and Arcamone, F., Doxorubicin Anticancer Antibiotics (1981) New York: Academic Press.

The term, "Active Ingredient" refers to the Survivin ASO compound I described herein. "Active Ingredient" also refers to a combination of a Survivin ASO and an additional anti-cancer agent, namely gemcitabine HCl.

As used herein, the term "patient" refers to a mammal that is afflicted with one or more disorders associated with Survivin expression or overexpression. It will be understood that the most preferred patient is a human. It is also understood that this invention relates specifically to the inhibition of mammalian Survivin expression or overexpression.

The term "pharmaceutically-acceptable salt" as used herein, refers to a salt of a compounds described herein. It should be recognized that the particular counterion forming a part of any salt of this invention is usually not of a critical nature, so long as the salt as a whole is pharmacologically acceptable and as long as the counterion does not contribute undesired qualities to the salt as a whole.

The terms "treatment" and "treating" are intended to refer to all processes wherein there may be a slowing, interrupting, arresting, controlling, or stopping of the progression of the disorders described herein, but does not necessarily indicate a total elimination of all symptoms.

As used herein, the term "effective amount" refers to the amount or dose of a compound, upon single or multiple dose administration, either alone or in combination with another anti-cancer agent, that is effective in treating the disorders described herein.

The term "therapeutically effective interval" is a period of time beginning when one of either (a) the Survivin ASO or (b) an additional anti-cancer agent is administered to a patient and ending at the limit of the anti-cancer beneficial effect of the combination of (a) and (b).

The term "therapeutically effective combination", used in the practice of this invention, means administration of (a) a Survivin ASO and (b) an additional anti-cancer agent, either simultaneously or separately, in any order. Thus, the term "therapeutically effective combination" refers to the administration of (a) Survivin ASO and (b) gemcitabine HCl, either simultaneously or separately, in any order.

When administered separately, the Survivin ASO and the additional anti-cancer agent may be administered on a different schedule. Either one may be administered before the other as long as the time between the two administrations falls within a therapeutically effective interval. The methods of administration of the Survivin ASO and the additional anti-cancer agent may vary. Thus, one agent may be administered orally, while the other is administered intravenously. It is possible that one of the products may be administered as a continuous infusion while the other is provided in discreet dosage forms. It is particularly important that the anti-cancer drug be given in the manner known to optimize its performance.

The anti-cancer agents are generally mixed with a carrier, which may act as a diluent, or excipient. The anti-cancer agents may be administered in the form of tablets, pills, powders lozenges, sachets, cachets, elixirs, suspensions, emulsion, solution, syrups or aerosols. Sterile injectable solutions may also be used.

The Survivin ASO and the additional anti-cancer agent used in the composition and method of the invention are often advantageously used in the form of salt derivatives, which are an additional aspect of the invention. When compounds of the invention possess an acidic group(s) or other reactive group, salts may be formed which are more water soluble and/or physiologically suitable than the parent compound in its acid form. Representative pharmaceutically acceptable salts include the alkali and alkaline earth salts such as lithium, sodium, potassium, calcium, magnesium, and aluminum.

Sodium salts are particularly preferred forms of the Survivin ASO compounds described herein. Salts are conveniently prepared from the free acid by treating the acid form in solution with a base or by exposing the acid to an ion exchange resin. Preferably, the Survivin ASO is prepared initially as an ammonium salt in solution. This is followed by conversion of the ammonium salt to the sodium salt via ion exchange or reverse phase chromatography, and/or by diafiltration with sodium salts on an ultrafiltration membrane.

Included within the definition of pharmaceutically acceptable salts are the relatively non-toxic, inorganic and organic base addition salts of compounds of the present invention, for example, ammonium, quaternary ammonium, and amine cations derived from nitrogenous bases of sufficient basicity to form salts with the compounds of this invention (see, for example, S. M. Berge, et al., "Pharmaceutical Salts," J. Phar. Sci., 66: 1-19 (1977)). Certain compounds of the invention may possess one or more chiral centers and may thus exist in optically active forms. All such stereoisomers as well as the mixtures thereof are intended to be included in the invention. If a particular stereoisomer is desired, it can be prepared by methods well known in the art, for example, by using stereospecific reactions with starting materials that contain the asymmetric centers and are already resolved or, alternatively, by methods that lead to mixtures of the stereoisomers and subsequent resolution by known methods. For example, a racemic mixture may be reacted with a single enantiomer of some other compound. This changes the racemic form into a mixture of diastereomers. Then, because the diastereomers have different melting points, different boiling points, and different solubilities, they can be separated by conventional means, such as crystallization.

The compositions of the present invention comprise a combination of therapeutically effective amounts of a Survivin ASO, described herein, and of the anti-cancer agents noted herein. The compositions can be administered by a variety of routes. In effecting treatment of a patient afflicted with disorders described herein, a composition of the present invention can be administered in any form or mode that makes the active ingredient bioavailable in an effective amount, including oral and parenteral routes. For example, compositions of the present invention can be administered orally, by inhalation, or by the subcutaneous, intramuscular, intravenous, transdermal, intranasal, rectal, occular, topical, sublingual, buccal, or other routes. Oral administration is generally preferred for treatment of the disorders described herein. However, oral administration is not the only preferred route. For example, the intravenous route may be preferred as a matter of convenience or to avoid potential complications related to oral administration. When the composition is administered through the intravenous route, an intravenous bolus or slow infusion is preferred.

An effective amount of the active ingredient can be readily determined by the attending diagnostician, as one skilled in the art, by the use of known techniques and by observing results obtained under analogous circumstances. In determining the effective amount or dose of compound administered, a number of factors are considered by the attending diagnostician, including: the species of mammal; its size, age, and general health; the specific neoplasm involved; the degree of or involvement or the severity of the neoplasm; the response of the individual patient; the particular compound administered; the mode of administration; the bioavailability characteristics of the preparation administered; the dose regimen selected; the use of concomitant medication; and other relevant circumstances.

Optimum dosages may vary depending on the relative potency of the active ingredient. In general, dosages range from 0.01 µg to 100 g per kilogram of body weight, and may be given once or more daily, weekly, monthly, or yearly. Persons of skill in the art can determine repetition rates for dosing, based on measured residence times and concentrations of the active ingredient in fluids or tissues. Following treatment with a single dose, it may be desirable to have the patient undergo maintenance therapy, wherein the active ingredient is administered in maintenance doses ranging from 0.01 µg to 100 g per kilogram of body weight, and may be given once or more daily, weekly, monthly, or yearly.

The following examples illustrate the methods of the present invention as well as the compounds and compositions employed to demonstrate the principles of the invention. The reagents and starting materials are readily available to one of ordinary skill in the art.

### EXAMPLES

### Example 1

The following compounds are representative of the Survivin ASO compound and the additional anti-cancer agents useful in the methods and compositions of the present invention.

### Compound I (Survivin ASO)

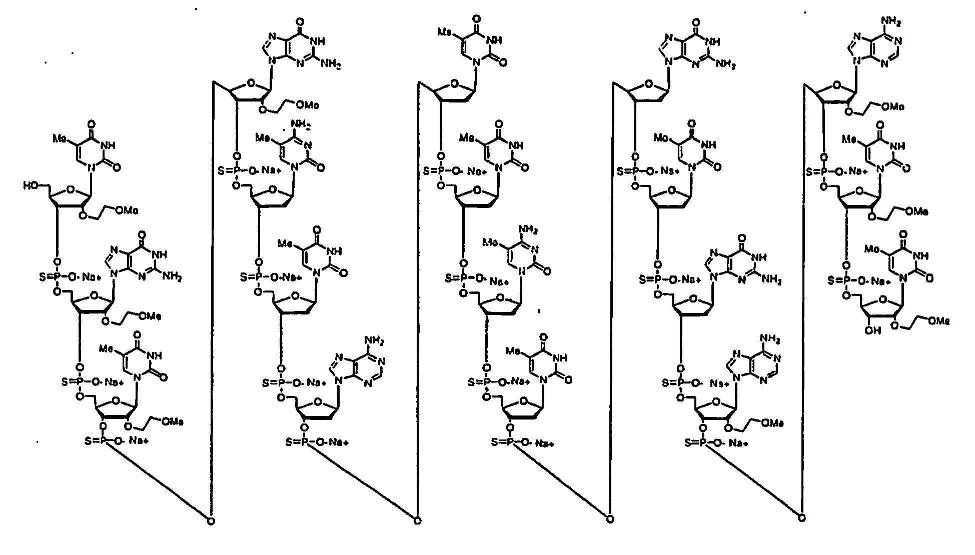

Compound I is a Survivin ASO, useful for inhibition of survivin expression or overexpression. Compound I may be prepared by following recognized general procedures as described in United States Patent Nos. 6,077,709; 6,165,788; and 6,335,194.

### Compound II (Gemcitabine HCl)

### 2'-deoxy-2',2'-difluorocytidine monohydrochloride (β-isomer); or 2',2'-difluoro-2'-deoxycytidine monohydrochloride; or 1-(4-amino-2-oxo-1H-pyrimidin-1-yl)-2-desoxy-2',2'-difluororibose

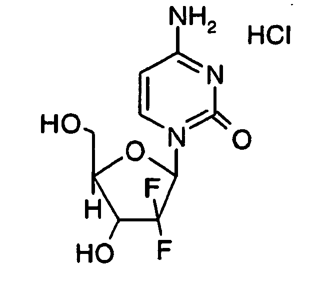

Gemcitabine HCl is described in US Patent No. 5,464,826, which is incorporated herein by reference for its disclosure of the methods of preparing the compound, formulating the compound, and the treatment of cancer using the compound.

### Assay Example 1

The cell based caspase 3 assay described herein is commonly used to evaluate anti-cancer agents, and has been described previously (*See* Carrasco et al., BioTechnique (2003) 34:1064-1067). The assay is performed as described below.

### Methods

HeLa cells are obtained from ATCC, Manassas, VA and are cultured in DMEM with L-Glutamine medium (Gibco/Invitrogen) supplemented with 10% Fetal Bovine Serum (Hyclone), 0.1 mM Non-Essential Amino Acids, and 1 mM Sodium Pyruvate (Gibco/ Invitrogen). For the caspase-3 assay, 96-well plates are plated with 1X10⁴ cells/well. Lipofectin (GIBCO/Invitrogen) transfection reagent is used at a concentration of 3µL/ml OPTIMEM reduced serum medium (Gibco/Invitrogen)/100nM Compound I. Lipofectin reagent is incubated with OPTIMEM medium for 30 minutes prior to the addition of Compound I. Fifty (50) nM of Compound I or its mismatch control (MM control) oligonucleotide is added and mixed. Cells are washed twice with IX phosphate buffered saline and then treated with the Compound I/Lipofectin mixture in OPTIMEM. After a 4 hr incubation period, OPTIMEM medium is replaced with complete growth medium. After 24 hours of transfection, either 10 nM of gemcitabine HCl or 1.25 nM of paclitaxel is added and incubated for an additional 48 hours. At the end of the incubation period, 3X lysis buffer (150 mM HEPES pH7.4, 150 mM HEPES pH 7.4, 450 mM NaCl, 150 mM KCI, 30 mM MgCl2, 1.2 mM EGTA, 1.5% NP40, 0.3% CHAPS, 30% Sucrose, 30 mM DTT, and 3.0 mM PMSF) containing 150 uM caspase-3 substrate Ac-DEVD-AMC (Biomol) is added in each well (50 µL/well) and incubated at 37°C for 1 hour. Caspases-3 activity is measured by reading proteolytically released fluor from the Ac-DEVD-AMC substrate using a plate-reading fluorometer with excitation at 360 nm and emission at 460 nm. After subtracting the plate background, the relative fluorescence units (RFU) are compared to untreated control and expressed as percentage increase in caspase-3 activity.

### Results

To investigate whether down-regulation of Survivin expression sensitizes HeLa cells to chemotherapy, combination treatment with Compound I and either gemcitabine HCl, doxorubicin HCl, or paclitaxel is evaluated. HeLa cells are transfected with Compound I or MM control oligonucleotide for 24 hours and treated with either 10 nM gemcitabine HCl or 1.25 nM of paclitaxel for an additional 48 hours. At the end of the incubation period, casapse-3 activity is measured. Data in Table 1 show that tumor cells treated with Compound I in combination with either gemcitabine HCl or paclitaxel produces greater than additive caspase-3 enzymatic activity versus either agent alone. Data are expressed as percentage caspase-3 activity compared to untreated control. "S.E.M" refers to standard error of the mean. MM control oligonucleotide treatment does not sensitize tumor cells to either gemcitabine HCl or paclitaxel.

**Table 1**

| **Treatment Group** | **Mean (% Caspase-3 Activity)** | **S.E.M.** |
|---|---|---|
| Gemcitabine HCl (10 nM) | 24.33 | 2.04 |
| MM Control (50 nM) | 8.66 | 4.75 |
| Compound I (50 nM) | 29.01 | 3.45 |
| MM Control (50 nM) + Gemcitabine HCl (10 nM) | 9.12 | 2.92 |
| Compound I (50 nM) + Gemcitabine HCl (10 nM) | 78.13 | 13.63 |
| Paclitaxel (1.25 nM) | 12.77 | 3.49 |
| MM Control (50 nM) + Paclitaxel (1.25 nM) | -5.79 | 1.90 |
| Compound I (50 nM) + Paclitaxel (1.25 nM) | 95.33 | 8.46 |

### Assay Example 2

The use of human xenograft tumor models has been described previously and is well known in the art. The following tumor types can be used to conduct the assay as described below. U-87 MG human glioblastoma are obtained from ATCC (Manasas, VA, USA) (Kiaris et al., Neoplasia (2000) 2(3):242-50).

### Methods

For xenograft studies, just before implantation, animals are irradiated (450 TBI (total body irradiation)), and cells are mixed in matrigel (1:1). A total of 6 X 10⁶ U-87MG tumor cells in a 0.2 mL volume are injected subcutaneously (s.c.) in the left rear flank. The treatment is initiated when the tumor volume reaches approximately 100 mg with an initial loading dose of 50 mg/kg of Compound I or MM control oligonucleotide each. All subsequent intravenous doses (25 mg/kg) of Compound I or MM control oligonucleotide are administered every other day. The treatment with sub-optimal doses of gemcitabine HCl, doxorubicin HCl, or paclitaxel is initiated one day following the loading dose. Gemcitabine HCl is administered intraperitoneally at 2.5 mg/kg every third day for a total of four doses. Paclitaxel is administered intravenously at 1 mg/kg every fourth day for a total of four doses.

Bi-dimensional measurements are performed twice per week, and tumor volumes are calculated based on the following formula: (Tumor Volume) = [(Length)(Width²)(II/6)]. Tumor volume data are transformed to a log scale to equalize variance across time and treatment groups. The data are analyzed using a two-way analysis of variance by time and treatment using SAS PROC MIXED software (SAS Institute, Inc., Cary, N.C.). The preferred correlation model for the repeated measures is AR (Auto Regressive of order 1).

Treatment groups are compared at each time point. The data are plotted as means and standard errors for each treatment group versus time. The presence of synergy in combination therapies is assessed on the tumor growth delay (TGD) scale. The time to reach a specified tumor size (1500 or 2000 or 2500 mg in this study) is determined for each animal. Tumors that do not reach that size are included in the analysis as right-censored values. Maximum likelihood analysis assuming a Weibull distribution is used to calculate mean times and standard errors for each treatment group. Tumor growth delay is the difference in mean times between each of the treated groups and the control group. A combination therapy is determined to have a synergistic effect if its TGD is significantly more than the sum of the TGDs for the individual therapies.

### Results

In Table 2, data show the effect of combination of Compound I with gemcitabine HCl. The combination causes a statistically significant (p = 0.0092) delay in tumor growth compared to either compound alone. Thus, the combination of Compound I with gemcitabine HCl results in a greater-than-additive delay in tumor growth of 3 to 4.6 days.

**Table 2**

| Tumor Growth Delay at: | 1500 mg | 2000 mg | 2500 mg |
|---|---|---|---|
| **Group** | **Mean ± S.E.M. (days)** | **Mean ± S.E.M. (days)** | **Mean ± S.E.M. (days)** |
| Saline | 0.0±0.9 | 0.0±0.6 | 0.0±0.6 |
| Gemcitabine HCl | 0.3+1.1 | 0.3+1.2 | 0.6+1 1 |
| MM Control | -0.6 ±0.9 | -0.2 ±1 1 | 0.4 ±0.9 |
| MM Control + Gemcitabine HCl | 1.1±0.9 | 1.5±0.9 | 1.9±0.8 |
| Compound I | 6.3±0.7 | 8.9±0.7 | 8.8 ± 0.5 |
| Compound I + Gemcitabine HCl | 10.9±0.8 | 12.2±0.9 | 14± 0.6 |
| Interaction Effect, Compound I + Gemcitabine HCl | 4.3 | 3 | 4.6 |

## Claims

1. Use of Compound I 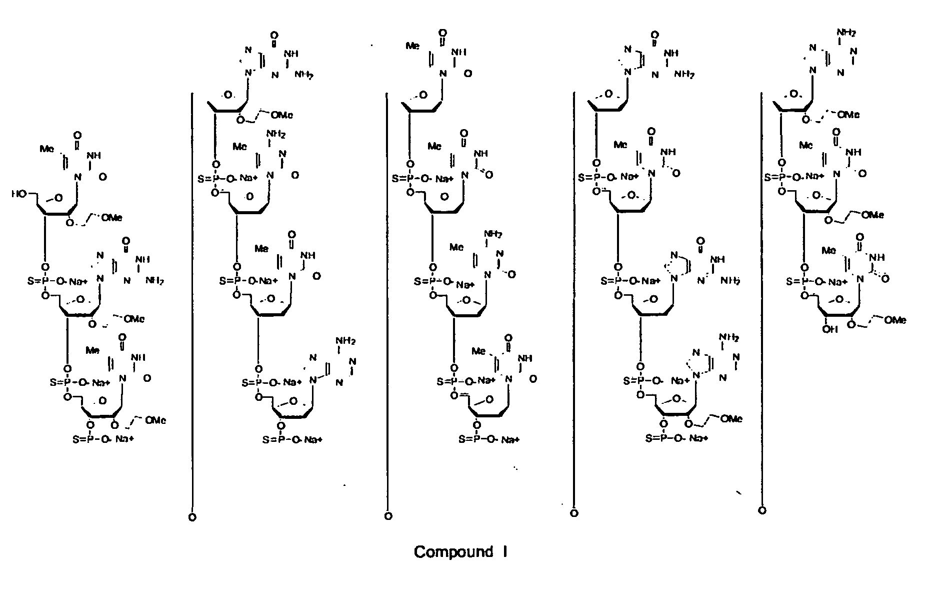 in the manufacture of a medicament for use in combination therapy for treating a susceptible neoplasm in a patient wherein said medicament is to be administered in combination with gemcitabine hydrochloride.

2. Use according to claim 1, wherein said Compound I is administered simultaneously with administration ofsaid gemcitabine hydrochloride.

3. Use according to claim 1, wherein said Compound I and said gemcitabine hydrochloride are administered separately, in any order, within a therapeutically effective interval.

4. Use according to any one of claims 1-3, wherein said combination therapy is via the parenteral route.

5. Use according to claim 4, wherein said parenteral route is via int ravenous administration.

6. Use according to claim 5, wherein said intravenous administration is via slow infusion.

7. Use according to any one of claims 1-6, wherein each of said Compound I and said gemcitabine hydrochloride is in the form of a sterile injectable solution.

8. Use according to any one of claims 1 -7, wherein said susceptible neoplasm is a cancer selected from the group consisting of pancreatic cancer, lung cancer, breast cancer, ovarian cancer, bladder cancer, hepatocellular cancer, colorectal cancer, and lymphoma.

9. Use according to claim 8, wherein said susceptible neoplasm is hepatocellular cancer.

10. A product containing Compound I 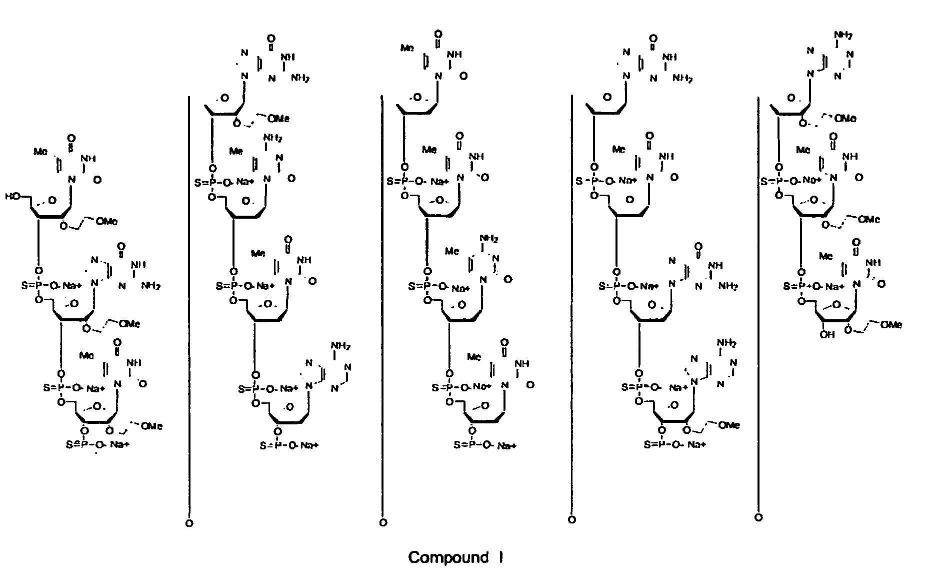 and gemcitabine hydrochloride as a combined preparation for the simul taneous or separate use in treating a susceptible neoplasm.

11. A product according to claim 10, wherein each ofsaid Compound I and said gemcitabine hydrochloride is in the form of a sterile injectable solution.

## Patentansprüche

1. Verwendung einer Verbindung der Formel I 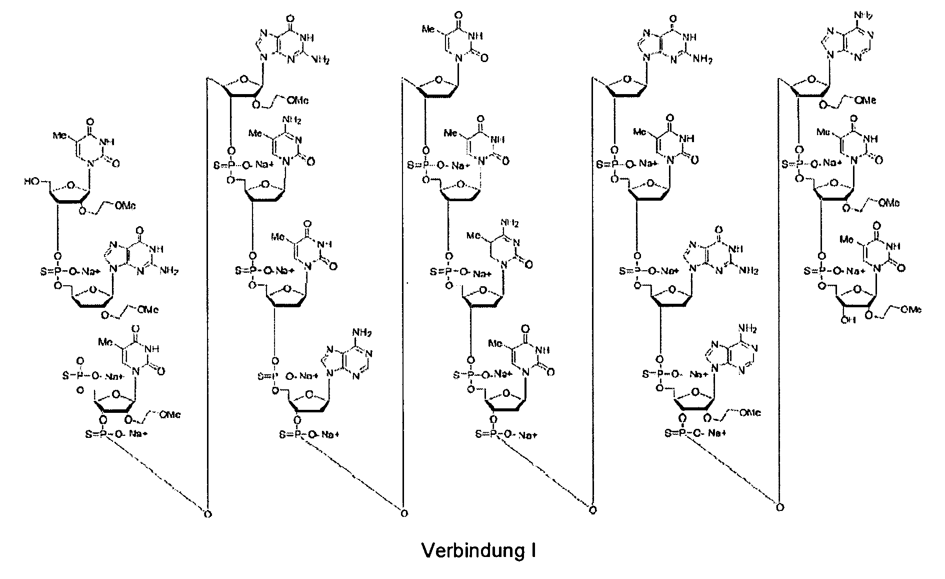 zur Herstellung eines Arzneimittels für eine Verwendung bei einer Kombinationstherapie für die Behandlung eines suszeptiblen Neoplasmas bei einem Patienten, worin dieses Arzneimittel in Kombination mit Gemcitabinhydrochlorid zu verwenden ist.

2. Verwendung nach Anspruch 1, worin die Verbindung I simultan mit einer Verabreichung von Gemcitabinhydrochlorid verabreicht wird.

3. Verwendung nach Anspruch 1, worin die Verbindung I und das Gemcitabinhydrochlorid separat in irgendeiner Reihenfolge innerhalb eines therapeutisch wirksamen Intervalls verabreicht werden.

4. Verwendung nach einem der Ansprüche 1 bis 3, worin die Kombinationstherapie auf parenteralem Weg angewandt wird.

5. Verwendung nach Anspruch 4, worin der parenterale Weg eine intravenöse Verabreichung ist.

6. Verwendung nach Anspruch 5, worin die intravenöse Verabreichung eine langsame Infusion ist.

7. Verwendung nach einem der Ansprüche 1 bis 6, worin die Verbindung I und das Gemcitabinhydrochlorid jeweils in der Form einer sterilen injizierbaren Lösung vorliegen.

8. Verwendung nach einem der Ansprüche 1 bis 7, worin das suszeptible Neoplasma ein Krebs ist, der aus der Gruppe ausgewählt ist, die besteht aus Pankreaskrebs, Lungenkrebs, Brustkrebs, Ovarialkrebs, Blasenkrebs, Hepatozellularkrebs, Colorektalkrebs und Lymphom.

9. Verwendung nach Anspruch 8, worin das suszeptible Neoplasma ein Hepatozellularkrebs ist.

10. Produkt mit einem Gehalt an einer Verbindung der Formel I 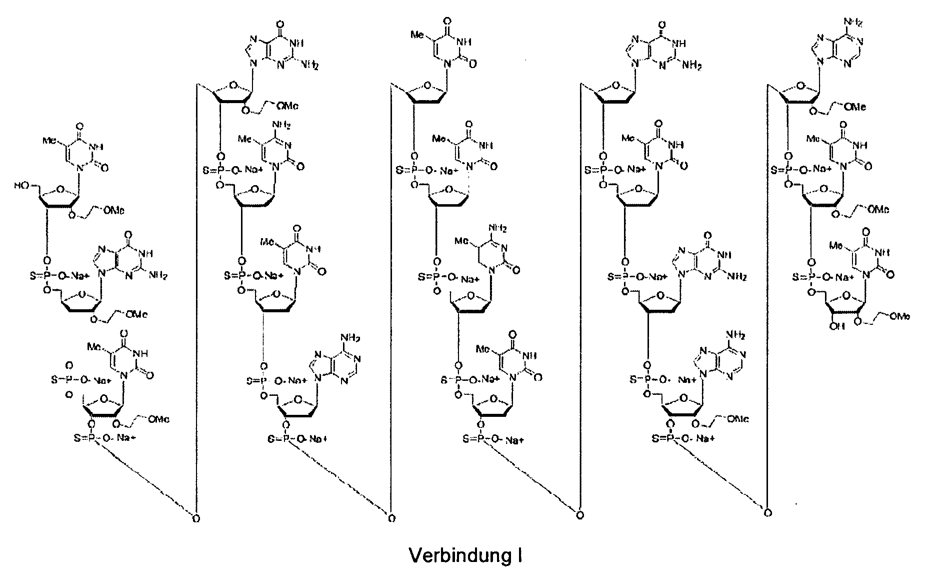 und an Gemcitabinhydrochlorid als ein Kombinationspräparat zur simultanen oder separaten Verwendung für die Behandlung eines suszeptiblen Neoplasmas.

11. Produkt nach Anspruch 10, worin die Verbindung I und das Gemcitabinhydrochlorid jeweils in der Form einer sterilen injizierbaren vorliegen.

## Revendications

1. Utilisation d'un composé I 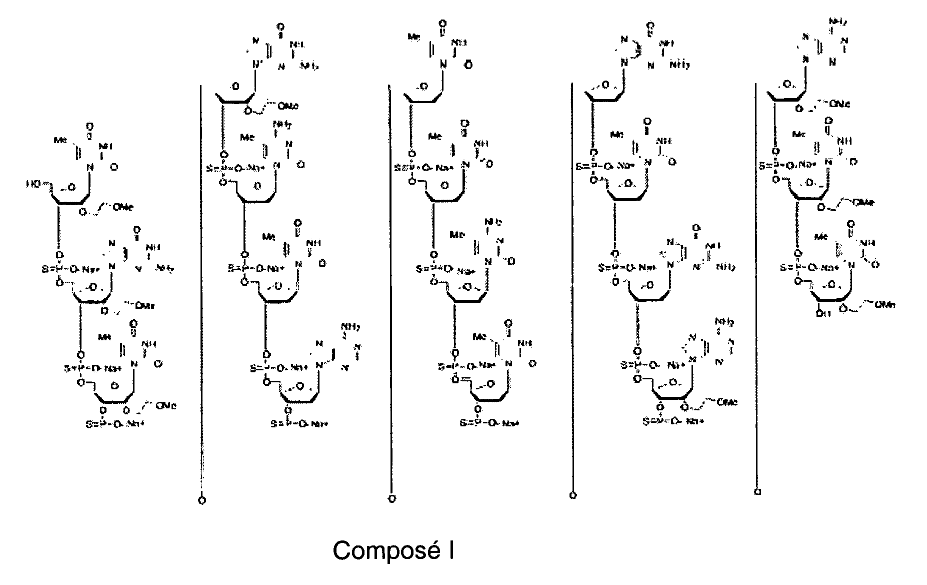 dans la fabrication d'un médicament pour une utilisation dans une thérapie en combinaison pour le traitement d'un néoplasme sensible chez un patient dans laquelle ledit médicament doit être administré en combinaison avec du chlorhydrate de gemcitabine.

2. Utilisation selon la revendication 1, dans laquelle ledit composé 1 est administré simultanément à l'administration dudit chlorhydrate de gemcitabine.

3. Utilisation selon la revendication 1, dans laquelle ledit composé I et ledit chlorhydrate de gemcitabine sont administrés séparément, dans n'importe quel ordre, dans un intervalle thérapeutiquement efficace.

4. Utilisation selon l'une quelconque des revendications 1 à 3, dans laquelle ladite thérapie en combinaison est via la voie parentérale.

5. Utilisation selon la revendication 4, dans laquelle ladite voie parentérale est via une administration intraveineuse.

6. Utilisation selon la revendication 5, dans laquelle ladite administration intraveineuse est via une perfusion lente.

7. Utilisation selon l'une quelconque des revendications 1 à 6, dans laquelle chacun dudit composé I et dudit chlorhydrate de gencitabine est sous la forme d'une solution injectable stérile.

8. Utilisation selon l'une quelconque des revendications 1 à 7, dans laquelle ledit néoplasme sensible est un cancer choisi dans le groupe constitué par un cancer pancréatique, un cancer du poumon, un cancer du sein, un cancer ovarien, un cancer de la vessie, un cancer hépatocellulaire, un cancer colorectal, et un lymphome.

9. Utilisation selon la revendication 8, dans laquelle ledit néoplasme sensible est un cancer hépatocellulaire.

10. Produit contenant le composé I 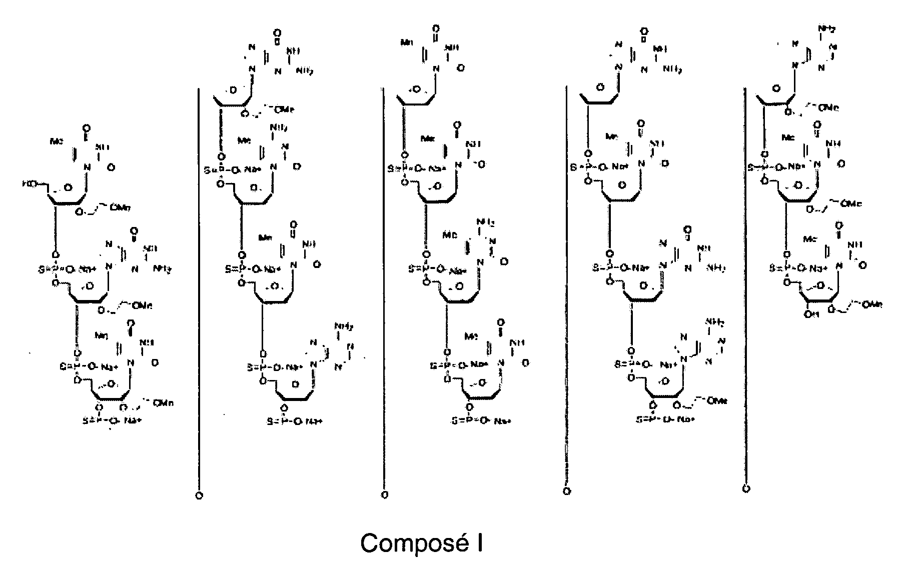 et du chlorhydrate de gemcitabine sous forme d'une préparation combinée pour l'utilisation simultanée ou séparée dans le traitement d'un néoplasme sensible.

11. Produit selon la revendication 10, dans lequel chacun dudit composé 1 et dudit chlorhydrate de gemcitabine est sous la forme d'une solution injectable stérile.


### SEQUENCE LISTING

<110> Eli Lilly and company
<120> Combination Therapy for the Treatment of cancer
<130> X-16613
<150> 60/564540
   <151> 2004-04-22
<160> 1
<170> PatentIn version 3.2
<210> 1
   <211> 18
   <212> DNA
   <213> Artificial Sequence
<220>
   <223> Synthetic construct
<400> 1
   tgtgctattc tgtgaatt 18